(19) Europäisches Patentamt
European Patent Office
Office européen des brevets

(11) **EP 1 259 810 B1**

(12) **EUROPÄISCHE PATENTSCHRIFT**

(45) Veröffentlichungstag und Bekanntmachung des Hinweises auf die Patenterteilung:
**02.11.2006 Patentblatt 2006/44**

(51) Int Cl.:
***G01N 33/557*** *(2006.01)* ***G01N 33/543*** *(2006.01)*

(21) Anmeldenummer: **01915204.0**

(22) Anmeldetag: **07.02.2001**

(86) Internationale Anmeldenummer:
**PCT/EP2001/001333**

(87) Internationale Veröffentlichungsnummer:
**WO 2001/057520 (09.08.2001 Gazette 2001/32)**

(54) **ANALYTISCHES MESS- UND AUSWERTEVERFAHREN FÜR MOLEKULARE INTERAKTIONEN**

ANALYTICAL MEASURING AND EVALUATION METHOD FOR MOLECULAR INTERACTIONS

PROCEDE ANALYTIQUE DE MESURE ET D'EVALUATION D'INTERACTIONS MOLECULAIRES

(84) Benannte Vertragsstaaten:
**AT BE CH CY DE DK ES FI FR GB GR IE IT LI LU MC NL PT SE TR**

(30) Priorität: **07.02.2000 DE 10005301**

(43) Veröffentlichungstag der Anmeldung:
**27.11.2002 Patentblatt 2002/48**

(73) Patentinhaber: **Biacore AB**
**754 50 Uppsala (SE)**

(72) Erfinder: **Trutnau, Hans-Heinrich**
**35619 Braunfels (DE)**

(74) Vertreter: **Widén, Björn et al**
**Biacore AB**
**Rapsgatan 7**
**754 50 Uppsala (SE)**

(56) Entgegenhaltungen:
• **MYSZKA DAVID G ET AL: "Equilibrium analysis of high affinity interactions using BIACORE." ANALYTICAL BIOCHEMISTRY, Bd. 265, Nr. 2, 15. Dezember 1998 (1998-12-15), Seiten 326-330, XP002201263 ISSN: 0003-2697**
• **HALL DAMIEN R ET AL: "Use of a resonant mirror biosensor to characterize the interaction of carboxypeptidase A with an elicited monoclonal antibody." ANALYTICAL BIOCHEMISTRY, Bd. 244, Nr. 1, 1997, Seiten 152-160, XP002201264 ISSN: 0003-2697**

**Beschreibung**

**1. Anwendungsgebiet**

Die Erfindung bezieht sich auf ein Meß- und Auswerteverfahren zur Bestimmung von Interaktionsparametem zwischen einem Analyt und einem Ligand wie unter anderem Geschwindigkeitskonstanten oder Bindungspartner-Aktivitäten. Die Aufnahme der Meßwerte erfolgt z. B. mit Biosensoren, in denen ein i. allg. immobilisierter erster Bindungspartner (Ligand L) mit einem zweiten Bindungspartner (Analyt A) versetzt wird und die Bildung des Ligand-Analyt-Komplexes LA im zeitlichen Verlauf erfaßt wird.

**2. Technischer Hintergrund**

Sogenannte Biosensoren zur Erfassung der zeitlichen Verläufe der Bildung von Analyt-Ligand-Komplexen sind in den unterschiedlichsten Ausführungen bekannt, wobei auch andere Vorrichtungen wie u. a. Array-Systeme zur Erfassung derartiger Reaktionen verwendbar sind. Dabei werden sowohl die zeitlichen Verläufe der Assoziation des Analyten am Liganden erfaßt, wenn die Analytkonzentration durch die Zugabe einer Analytlösung bestimmter Konzentration erhöht wird, sowie die Dissoziation bei Zugabe einer geringer konzentrierten Lösung oder mit einer Konzentration von Null. I. allg. wird der zeitliche Verlauf der Komplexbildung mit der Funktion R(t) oder $R_t$ beschrieben, wobei die Konzentration des Komplexes bzw. seine zeitliche Änderung mit $c_t(LA)$ bzw. $dc_t(LA)/dt$ bezeichnet wird. In erster Näherung wird für den zeitlichen Verlauf der Funktion R ein exponentieller Verlauf erster Ordnung mit dem Exponenten $k_{on}$ bei der Assoziation und $k_{off}$ bei der Dissoziation des Komplexes angenommen. Zur Erfassung dieser Werte ist es bisher üblich, die Reaktion, z. B. die Komplexbildung, teilweise oder bis zum Erreichen eines Gleichgewichtswertes $R_{eq}$, bei dem die Assoziation des Komplexes aus Analyt und Ligand im Gleichgewicht steht mit dessen Dissoziation, auszumessen. Es folgt eine Dissoziation des Komplexes und eine Regenerations- und Waschphase. Dies ist ein sehr zeitaufwendiges Verfahren, insbesondere wenn hintereinander mehrere Messungen mit unterschiedlichen Konzentrationsänderungen vorgenommen werden sollen. Eine weitere Auswertung der erhaltenen Exponenten führt zu den eigentlich interessierenden kinetischen Geschwindigkeitskonstanten, das Verfahren bedarf jedoch einer konstanten Analytkonzentration.

Bei den bisherigen Auswertungsverfahren der Meßwerte wird meistens davon ausgegangen, daß die Konzentration des zu dem Liganden zugegebenen Analyten trotz der Komplexbildung konstant ist. Dies wird z. B. dadurch angenähert, daß die Analytkonzentration vielfach höher ist als die Ligandkonzentration oder durch ständigen Austausch im Durchflußsystem. Tatsächlich jedoch tritt z. B. bei der Assoziation im Küvettensystem immer eine Verarmung des Analyten oder eine Konzentrationsänderung auf, die dazu führt, daß der Gleichgewichtswert $R'_{eq}$, der tatsächlich erreicht wird, sich von dem hypothetischen Wert $R_{eq}$ bei konstanter Analytkonzentration unterscheidet. Eine Analyse der Meßwerte mit einer Näherungsfunktion zweiter Ordnung ergibt zwar einen genaueren Wert des Koeffizienten der Exponentialfunktion, ist aber aufwendig und bedarf der zusätzlichen Ermittlung bzw. Berücksichtigung einer Reihe von Randbedingungen des Tests.

Zur Konzentrationsbestimmung werden initiale Raten herangezogen. Die initialen Raten werden dadurch erhalten, daß in den Anfang der Assoziationskurve eine Ausgleichsgerade gelegt wird, deren Steigung die initiale Rate aber unterbewertet, da die Gerade die Krümmung der Kurve nicht berücksichtigt. Desweiteren ist es bekannt, daß beim Auftragen der initialen Raten in einem Diagramm über die Konzentration des Analyten c(A), bei mehrmaliger Messung mit jeweils unterschiedlichen Konzentrationen, theoretisch eine Gerade durch den Ursprung mit der Steigung $R_{max}$ * $k_{ass}$ erhalten wird, wobei $R_{max}$ die maximal mögliche Reaktion z. B. des Biosensors auf die Zugabe eines Analyten im Überschuß darstellt. Die Steigung dieser Geraden ist aber durch die o.g. Unterbewertung ebenfalls verfälscht, so daß diese Art von Diagramm nicht zur Bestimmung von $k_{ass}$ herangezogen wird.

Zur Erfassung der Meßwerte werden u. a. Biosensoren nach dem Durchflußprinzip oder dem Küvettenprinzip verwendet, wobei die unterschiedlichsten Meßverfahren im Stand der Technik bekannt sind. Bei Durchflußsystemen wird eine Sensoroberfläche, auf der der Ligand immobilisiert ist, pro Analyse mit einem konstanten Strom einer Analytlösung beaufschlagt. Hierbei ist die Näherung einer vorgewählten konstanten Analytkonzentration im Prinzip gegeben. Bei Küvettensystemen wird eine Meßzelle mit einer Analytlösung gefüllt und die Reaktion mit dem Liganden an einer Sensoroberfläche erfaßt. Besonders hier tritt eine Verfälschung der Meßergebnisse auf, da bei tatsächlichen Reaktionen die Analytkonzentration verändert wird. Werden mehrere Meßvorgänge mit unterschiedlichen Konzentrationen hintereinander durchgeführt, so wird normalerweise die Küvette mit einer Pufferlösung gespült oder auf andere Weise regeneriert, und anschließend eine Lösung mit geänderter Konzentration eingebracht.

Bei mehrfachen Titrationen, d. h. Änderung der Konzentration, werden die Meßkurven bisher so lange aufgenommen, bis die Komplexbildung einen Gleichgewichtszustand erreicht hat. Es werden lediglich die Gleichgewichtszustände zur Bestimmung weiterer Größen, nicht jedoch der Kinetik oder initialer Raten, verwendet. Vielmehr wird bei solchen Mehrschritt-Titrationen ein Zugang zur Kinetik ausdrücklich ausgeschlossen.

So offenbaren z.B. Myszka D.G. et al., Anal Biochem. 265 (1998) 326-330, eine solche sukzessive Erhöhung der Analytkonzentration von außerhalb des Messgerätes (d.h. weit entfernt vom Messpunkt), die jedoch eine Gleichgewichtseinstellung nach jeder Konzentrationsänderung verlangt, was insgesamt bis zu mehr als 10 Stunden dauern kann und einen entsprechend hohen Probenverbrauch bedeutet. Zudem bewirkt der Wechsel einer Analyt-Lösung außerhalb des Gerätes eine deutliche Vermischung mit der vorauslaufenden Lösung auf dem Weg zum Messpunkt und damit eine lange andauernde Verzerrung der zeitlichen Messkurven. Daher können lediglich die finalen Gleichgewichtswerte hinsichtlich der allgemeinen Affinität bewertet, nicht jedoch die Messkurven bezüglich der gewünschten kinetischen Konstanten mittels eines kinetischen Fittings ausgewertet werden.

Für Durchfluß-Systeme sind sequentiell bzw. hintereinander angeordnete sogenannte Probenschleifen bekannt, die nacheinander befüllt und in die Meßkammer ausgespült werden können. Jedoch weisen die Probenschleifen jeweils ein produktionstechnisch aufwendiges exakt definiertes Volumen und zwischen ihnen ein undefiniertes Extravolumen, das auch als Totvolumen bezeichnet wird, auf. Im Totvolumen kann es zu unerwünschten Durchmischungen verschiedener Lösungen kommen. Die Schleifen sind weder von ihrer Auslaß-Seite her noch teilweise und auch nicht unabhängig voneinander befüllbar; und sie sind nicht unabhängig voneinander in die Meßkammer spülbar.

## 3. Darstellung der Erfindung

### a) Technische Aufgabe

Ausgehend vom Stand der Technik hat sich die Erfindung die Aufgabe gestellt, ein Verfahren anzugeben, mit dem die Meßzeiten bei der Erfassung der Meßwerte bei der Komplexbildung verkürzbar sind, die tatsächlichen Verhältnisse bei der Komplexbildung, d. h. Änderung der Analytkonzentration, berücksichtigt werden, und die Durchführung mehrerer Messungen hintereinander ohne aufwendige Spülvorgänge z. B. in einer Küvette möglich ist, sowie es möglich ist, die kinetischen Konstanten aus den initialen Raten, und unter idealen Verhältnissen auch aus den Kurvenexponenten zu ermitteln.

### b) Lösung der Aufgabe

Erfindungsgemäß wird diese Aufgabe durch die Verfahrensmerkmale des Anspruchs 1 und die Vorrichtungsmerkmale des Anspruchs 16 gelöst.

Vorteilhafte Ausgestaltungen der Erfindung sind jeweils Gegenstand von Unteransprüchen.

Der Kemgedanke der Erfindung besteht darin, daß die Änderung der Analytkonzentration sowohl bei der Assoziation als auch der Dissoziation des Komplexes berücksichtigt wird. Dadurch nimmt die Funktion R einen Gleichgewichtswert $R'_{eq}$ an, der sich vom Wert $R_{eq}$ unterscheidet. Bei der Assoziation, d.h. Zugabe eines Analyten erhöhter Konzentration zu dem Liganden, tritt während der Assoziation eine Verarmung des Analyten auf, die dazu führt, daß $R'_{eq}$ kleiner ist als der theoretische Wert $R_{eq}$. Im ersten Moment der Reaktion jedoch, d. h. wenn die Analytlösung z. B. in die Küvette eines Biosensors eingebracht wird, ist die initiale Rate der Funktion R unabhängig davon, ob im späteren Verlauf eine Analytverarmung durch Bindung des Analyten an den Liganden auftritt oder konstante Analytkonzentration angenommen wird. Zum Zeitpunkt t = 0 hat noch keine Reaktion zwischen Analyt und Ligand stattgefunden. Durch die Berücksichtigung des tatsächlichen Reaktionsverlaufs ändert sich auch der Exponentialkoeffizient von $k_{on}$ in $k'_{on}$. Tatsächlich jedoch bleibt die Beziehung zwischen den einzelnen Größen der Exponentialfunktion erhalten, d. h.:

$$k'_{on} * R'_{eq} = \frac{dR_{ass}(t=0)}{dt} \qquad (1)$$

und

$$k'_{off} * \Delta R' = \frac{dR_{diss}(t=0)}{dt} \qquad (2)$$

wobei $\Delta R'$ die Differenz zwischen dem Gleichgewichtswert der Dissoziation und dem Startwert $R_{st}$ der Funktion R ist.

Das bedeutet, daß aus den tatsächlichen Meßwerten z. B. die initiale Rate ermittelbar ist. Dies gilt ebenso, wenn die Exponentialfunktion zwar nach 1. Ordung, aber mit zwei Koeffizienten $k'_{on(1)}$ und $k'_{on(2)}$ verläuft, wobei sich die beiden korrespondierenden initialen Raten zu einer Gesamtrate addieren.

Dabei ist es möglich, daß die Meßkurve der Funktion R bis zum Erreichen des Gleichgewichtswertes $R'_{eq}$ aufgenommen wird, um z. B. mit Hilfe eines mathematischen Programms den Exponenten $k'_{on}$ oder $k'_{off}$ zu ermitteln und daraus die initialen Raten der Assoziation oder Dissoziation. Im Rahmen der Erfindung wird lediglich ein Teil der Kurve erfaßt, d.h. die Erfassung der Funktion R wird zu einem früheren Zeitpunkt abgebrochen, um aus einer Extrapolation des Kurvenabschnitts die Gleichgewichtslage zu ermitteln. Der Exponent k wird dabei mit einer non-linearen Näherung erhalten, kann aber auch über die Lineare Regression der Ableitung der Funktion R bestimmt werden.

Aus den initialen Raten können dann die jeweiligen Assoziations- und Dissoziationsgeschwindigkeitskonstanten $k_{ass}$ und $k_{diss}$ ermittelt werden gemäß:

$$\frac{dR_{ass}(t=0)}{dt} = k_{ass} * R_{max} * c_0(A) \tag{3}$$

im Falle der Komplexassoziation mit der Startkonzentration des Analyten $c_0(A)$ bzw.:

$$\frac{dR_{diss}(t=0)}{dt} = k_{diss} * (-R_{st}) \tag{4}$$

im Falle der Komplexdissoziation, wobei die Größe $R_{st}$ den Startwert der Funktion R bei der Dissoziation angibt. Die Startkonzentration $c_0(A)$ ist i. allg. durch den Versuch von außen vorgegeben.

Der Vorteil der Erfindung besteht darin, daß mehrmals hintereinander, z. B. in einer Küvette, eine Messung mit jeweils schrittweise veränderter Analytkonzentration durchführbar ist, wobei es nicht notwendig ist, daß jede Messung bis zum Erreichen des Gleichgewichtswertes zu Ende geführt wird, sondern bereits vorher abgebrochen werden kann bzw. die Konzentration des Analyten erhöht oder erniedrigt werden kann. Auch die Durchführung von Meßserien mit mehrmaliger auch abwechselnd aufeinanderfolgender Analyterhöhung und -verminderung zur Erfassung von Assoziations- und Dissoziationskurven ist möglich.

Obschon sich die Start-Raten von multiplen, i-fachen Assoziationsschritten aus der Summe der vorangegangenen und der neuen Assoziation bilden, lassen sich die Netto-Start-Raten der jeweiligen Assoziationsschritte basierend auf den eingangs formulierten Gleichungen (1) und (3) für die Assoziation ermitteln gemäß:

$$k'_{on,i} * (R'_{eq,ass,i} - R'_{st,ass,i}) = (dR_{ass,i}/dt)_{t=0} \tag{5}$$

und:

$$(dR_{ass,i}/dt)_{t=0} - (dR_{ass,i-1}/dt)_{t=st,ass,i} = (dR_{ass,i,net}/dt)_{t=0} \tag{6}$$

und auswerten gemäß:

$$(dR_{ass,i,net}/dt)_{t=0} / c_{0,i,net}(A) = -k_{ass} * R_{st,ass,i} + k_{ass} * R_{max} \tag{7}$$

was einer Geradengleichung entspricht und sich entsprechend auftragen lässt.

Der Quotient aus initialer Netto-Assoziationsrate und Analyt-Netto-Startkonzentration, aufgetragen gegen das Assoziations-Startsignal $R_{st,ass,i}$, liefert linear gefittet eine Gerade mit der Steigung = $-k_{ass}$ und einen x-Achsenabschnitt $R_{max}$. Die einzelnen Phasen multipler Dissoziationsschritte aus Küvettensystemen können direkt nach den Gleichungen (2) und (4) für die Dissoziation ausgewertet werden.

Die Berechnung von sogenannten Fit-Kurven, mit denen die erhaltenen Meßergebnisse angenähert oder im Falle des vorzeitigen Abbruchs der Messung extrapoliert werden, erfolgt mit mathematischen Annäherungsprogrammen, die eine Exponentialfunktion, auch eine Doppelt-Exponentialfunktion, erster oder höherer Ordnung ermitteln, die die Meßergebnisse mit größtmöglicher Genauigkeit annähert.

Die Berechnung einer derartigen Kurve kann durch die Auswertungseinheit z. B. eines Biosensors selbsttätig während der Messung erfolgen.

Die schrittweise Änderung der Konzentration in der Meßkammer kann zu beliebigen Zeitpunkten während der Aufnahme der Meßergebnisse erfolgen. Insbesondere wird jedoch die Halbwertzeit der Funktion R vorgeschlagen, zu der die Funktion, beispielsweise bei einer Assoziation die Hälfte ihres Endwertes $R_{eq}$ erreicht hat.

Wird die Ableitung der Funktion R(t) in einem Diagramm gegen die Funktion R(t) aufgetragen, so wird, wenn die aufgetragenen Werte z. B. mit einer linearen Regression genähert werden, eine Gerade erhalten, deren y-Achsenabschnitt gerade der initialen Rate entspricht, die sich auch, und in Übereinstimmung mit der Gleichung (1), aus dem absoluten Wert der Geradensteigung (-k'on) und dem x-Achsenabschnitt R'$_{eq}$ ergibt.

Zur Vereinfachung der Auswertung wird angenommen, daß die Reaktionen unter idealen Bedingungen ablaufen, d. h., daß insbeondere keine Analytverarmung auftritt. Dies kann z. B. bei Biosensoren mit Durchflußsystemen angenähert werden, indem ein stetiger Durchsatz der Lösung in der Meßkammer erfolgt. damit vereinfachen sich die Näherungsfunktionen, da z. B. eine Exponentialfunktion erster Ordnung für den Verlauf der Funktion R angenommen werden kann.

Gemäß obenstehender Formel (3) zur Bestimmung der Assoziationsgeschwindigkeitskonstante kann, wenn die anderen Größen bekannt sind, auch die Anfangskonzentration $c_0(A)$ einer neu in eine Meßkammer eingebrachten Lösung bestimmt werden. Dazu kann z. B. in einer vorhergehenden Versuchsreihe mit bekannten $c_0(A)$-Werten aus den ermittelten initialen Raten eine Kalibrationsgerade erstellt werden gemäß initiale Rate = f($c_0(A)$); der unbekannte $c_0(A)$-Wert einer Probe kann dann mit der aus den Meßwerten ermittelten initialen Rate aus der Kalibrationsgeraden berechnet werden.

Ebenso ist es, besonders unter Verwendung von Küvettensystemen, möglich, daß aus den aus einem Assoziationsvorgang erhaltenen Werten die Dissoziationsgeschwindigkeitskonstante erhalten wird und umgekehrt. Dies beruht darauf, daß z. B. bei einer Assoziation im ersten Moment, zu dem die Reaktion beginnt, noch keine Dissoziation erfolgt. Da die Funktion R aber einen Gleichgewichtswert annimmt, bei dem Assoziation und Dissoziation im Gleichgewicht stehen, enthält dieser Wert also auch Informationen über $k_{diss}$. Die Umkehrung dieses Ansatzes führt zu einem analogen Ergebnis für die Dissoziation. Die entsprechenden Formeln lauten:

$$k_{ass} = \frac{\dfrac{(dR_{diss}/dt)_{t=0}}{\Delta R_{eq-st\_diss}}}{\left(\dfrac{c_{eq'\_diss}(A)}{R_{eq'\_diss}}\right)_{c_{eq'\_diss}=0} \cdot R_{max}} \qquad (8)$$

wobei $\Delta R_{eq-st\_diss}$ der Differenz zwischen dem Endwert, der idealerweise 0 ist, und den Anfangswerten von R bei der Dissoziation entspricht, $C_{eq'\_diss}(A)$ die Konzentration von A nach der Dissoziation im Gleichgewicht ist und $R_{eq'\_diss}$ der Gleichgewichtswert von R nach der Dissoziation ist, und in den Nenner der Grenzwert für $c_{eq'\_diss} = 0$ eingesetzt wird, und:

$$k_{diss} = \frac{\dfrac{(dR_{ass}/dt)_{t=0}}{c_0(A)}}{\left(\dfrac{R_{eq'\_ass}}{c_{eq'\_ass}(A)}\right)_{R_{eq'\_ass}=0}} \qquad (9)$$

mit den entsprechenden Werten für die Assoziation.

Ebenso kann das Verfahren zur Auswertung bei anderen Affinitätsbiosensoren oder Array-Systemen in Lösung angewendet werden, sowie bei zeitaufgelösten ELISA- oder RIA-Techniken oder anderen festphasengestützten Systemen, die dem Stand der Technik entnehmbar sind.

Zur Realisierung einer ständig die Meßkammer durchströmenden Lösung wird in einem Küvettensystem die Konzentration der Lösung durch ständige Zugabe und Entnahme eines Teils der Lösung konstant gehalten. Dazu sind z. B. zwei Kanülen mit Lösungen vorhanden, mit denen entweder ständig frische Lösung in der Meßkammer umgewälzt wird, oder mit denen ein Teil der Lösung ständig entnommen und wieder zugeführt wird, um ein Durchflußsystem mit konstanter Konzentration zu simulieren.

In diesem Fall, wie auch in idealen Durchfluß-Systemen mit konstanter Konzentration der Lösung in der Meßzelle, wird die Funktion R durch eine Exponentialfunktion erster Ordnung angenähert und es gilt folgende Beziehung:

$$k'_{on,i} = k_{ass} * c_{0,i}(A) + k_{diss} \qquad (10)$$

wobei der Index i die i-te schrittweise Änderung der Konzentration in einem Mehrschritt-Experiment angibt. Nach diesem Verfahren lassen sich beide Geschwindigkeitskonstanten aus derselben Beziehung ermitteln.

Für ideale Durchfluß-Systeme mit konstant gehaltener Analyt-Startkonzentration während einer Assoziationsphase ist als bekannt gezeigt worden, daß die Startkonzentration genau dann gleich der Dissoziations-Gleichgewichtskonstante $K_D = k_{diss}/k_{ass}$ ist, wenn mit dieser Konzentration die Hälfte der immobilisierten Ligandenmoleküle mit Analytmolekülen gebunden werden, d.h. ½ $R_{max}$ erreicht wird. Für diesen Fall läßt sich darüber hinaus aber zeigen, daß die Assoziationsphase bis zur Erreichung des Gleichgewichtswertes gerade halb so lange dauert wie die sich anschließende Dissoziationsphase, um wieder auf den Ausgangswert Null zurückzukehren. Bei höheren Analyt-Startkonzentrationen verläuft die Assoziation schneller. Unter dieser Voraussetzung schneidet die Dissoziationskurve, wenn sie mit der vorausgelaufenen Assoziationskurve überlagert wird, diese Assoziationskurve exakt im Goldenen Schnitt des Assoziations-Gleichgewichtswertes, was dem Wert 0,618...* $R_{eq\_ass}$ entspricht. D.h. durch die Applikation verschiedener Analyt-Startkonzentrationen und eine darauf folgende Regression auf den oben beschriebenen Schnittmeßwert läßt sich der $K_D$-Wert extrapolieren. Umgekehrt muß eine Analyt-Startkonzentrationen, numerisch im $K_D$-Wert appliziert, den oben beschriebenen Schnittmeßwert ergeben; andernfalls verläuft die aufgezeichnete Interaktion nicht ideal. Prinzipiell erlaubt die Multi-Schritt-Kinetik einen analogen Ansatz.

Generell, insbesonders für Küvettensysteme mit variabler Analytkonzentration in der Meßzelle während eines Meßschrittes, gelten desweiteren die folgenden Beziehungen:

$$k'_{on} = k_{ass} * c'_{eqass}(A) + k'_{offmax} \qquad (11)$$

mit der Geradensteigung $k_{ass}$, wobei $c'_{eqass}(A)$ die Analytgleichgewichtskonzentration nach der Assoziation und $k'_{offmax}$ der Maximalwert des Koeffizienten der Exponentialfunktion bei der Dissoziation ist; und

$$k'_{off} = k_{diss} * (-R_{stdiss})/(R'_{eq} - R_{stdiss}) \qquad (12)$$

mit der Geradensteigung $k_{diss}$, wobei der Index stdiss den Startwert bei der Dissoziation bedeutet.

### c) Ausführungsbeispiele des Biosensors

Zur Durchführung des Verfahrens wird ein an sich bekannter Biosensor verwendet, der mit einer Sensoroberfläche ausgestattet ist, an der die Liganden immobilisiert d. h. gebunden sind. Diese Bindung kann z. B. wie im Stand der Technik bekannt über eine chemische Bindung des Liganden oder mit einem auf den Liganden abgestimmten Rezeptor erfolgen. Über der Sensoroberfläche ist eine Meßkammer oder Küvette ausgebildet, die mit einer Lösung des Analyten beaufschlagbar ist. Zur Erfassung der Komplexbildung, d. h. der Bindung des Analyten am Liganden, sowie deren zeitlicher Verlauf können verschiedene Meßverfahren verwendet werden, z. B. die Erfassung des Reflexionsvermögens der Rückseite des Sensors, das sich mit dem Grad der Komplexbildung c(LA) ändert. Zum Einbringen der Lösung in die Meßkammer dient eine Kanüle, die mit der Meßkammer verbunden ist und entweder die Lösung zugibt und/oder absaugt. Die Meßkammer kann auch mit zwei getrennten Kanülen zur Zugabe/Herausnahme ausgestattet sein. Auch die Beaufschlagung mit einer Spülflüssigkeit über eine der Kanülen ist möglich. Zur Durchmischung der Lösung in der Meßkammer, um Konzentrationsgradienten in der Lösung während der Reaktion zu vermeiden, ist eine Vorrichtung zur Durchmischung der Lösung vorhanden, dies kann z. B. ein Vibrationsrührer sein. Die Ausstattung des Biosensors mit einer vorzugsweise elektronischen Steuerung sowie geeigneten Meßgeräten nebst Datenerfassungssoftware ist dem Stand der Technik entnehmbar.

Erfindungsgemäß ist der Biosensor derart ausgebildet, daß die Lösung in der Meßkammer zumindest teilweise instantan mehrmals mit Lösungen unterschiedlicher Analytkonzentration austauschbar ist, wobei das Flüssigkeitsvolumen konstant gehalten wird. Das bedeutet, daß zumindest ein Teil der Lösung in der Meßkammer, oder die gesamte Lösung, entnommen und gleichzeitig eine neue Lösung mit einer anderen Konzentration zugegeben wird. Durch den Austausch zumindest eines Teils der Lösung durch eine andere Lösung mit gewünschter Konzentration wird die Gesamtkonzentration in der Meßkammer in definierter Weise verändert. Die Art des Austauschens der Flüssigkeit kann im Rahmen der Erfindung beliebig ausgeführt oder wie im folgenden dargelegt vorgenommen werden. In einem Durchflußsystem wird die komplette Lösung in der Meßkammer ausgetauscht, bei einem Küvettensystem zumindest ein Teil davon. Mit dem mehrmaligen Austauschen der Lösung wird eine schrittweise Änderung der Konzentration in der Meßkammer ermöglicht, um das erfindungsgemäße Verfahren durchführen zu können.

Der Vorteil des erfindungsgemäßen Biosensors besteht darin, daß z.B. bei Küvettensystemen das Volumen der Lösung konstant gehalten ist und damit die Anzahl der Parameter bei der mathematischen Auswertung der Meßergebnisse verringert wird. Der instantane Austausch der Lösungen führt zudem besonders bei Durchflußsystemen zu scharfen Übergängen zwischen einzelnen Assoziations- bzw. Dissoziationsphasen, was für die Bestimmung der initialen Raten notwendig ist.

Vorzugsweise erfolgt der Austausch zumindest eines Teils der Lösung in der Meßkammer über eine oder über mehrere Kanülen gleichzeitig. Insbesondere wird die Lösung mit einer Kanüle entnommen und mit einer anderen Kanüle eine neue Lösung mit einer beliebig wählbaren Konzentration zugegeben.

Werden die Kanülen getrennt voneinander an der Meßkammer angeordnet, z. B. einander gegenüberliegend, kann die Entnahme und Zuführung auch gleichzeitig erfolgen, wobei zum vollständigen Austausch der Lösung in einem Küvettensystem die Entnahme bzw. Zuführung derart erfolgt, daß die Meßkammer durchgespült wird, um das Verbleiben unerwünschter Rückstände zu vermeiden. Die Kanülen können auch koaxial angeordnet werden, d. h. eine verläuft in der anderen, wobei die innere entweder die Lösung entnimmt oder zuführt.

Zur Förderung der Lösungen durch die Kanülen in die Meßkammer hinein oder heraus sind Pumpen vorgesehen, mit denen die Menge der jeweils geförderten Lösung dosierbar ist. Dazu werden sogenannte Mikropumpen verwendet, die eine Dosierung mit der gewünschten Genauigkeit erlauben, um wohl definierte Mengen an Lösung der Meßkammer zu entnehmen oder ihr zuzuführen. Diese Pumpen sind unter anderem als Schlauchpumpen, Spritzenpumpen oder piezoelektrische Pumpen auslegbar, wobei die Betätigung mit Stellmotoren, elektrischen Antrieben etc. erfolgt und sie ebenfalls elektronisch angesteuert sind.

Die Durchmischung der Lösung in der Meßkammer kann auch dadurch erfolgen, daß mit einer oder mehreren Kanülen ein Teil der Lösung angesaugt und sofort wieder zurückgepumpt wird, so daß die Lösung ständig umgewälzt und durchmischt wird. Vorzugsweise erfolgt eine Umwälzung mit mindestens 1 $\mu$l/min, um eine homogene Konzentration der Lösung in der Meßkammer während der Komplexbildung zu gewährleisten.

Eine weitere vorteilhafte Ausgestaltung der Erfindung besteht darin, daß an der Meßkammer in bestimmter Weise zwei oder mehr sogenannte Probenschleifen beliebigen Volumens vorhanden sind, die manuell und/oder mittels eines Probengebers automatisch befüllt werden können. Vorzugsweise sind die Probenschleifen parallel angeordnet zwischen zwei Mehrfach-Wahlventilen, wobei diese gesamte Anordnung über ein Schaltventil in den Durchfluß einschaltbar ist. Die Probenschleifen können so außerhalb des Durchflusses einerseits über die Wahlventile unabhängig voneinander und andererseits über das Schaltventil von ihrem Ausflußende her ganz oder nur teilweise befüllt werden, um z. B. Analytlösung zu sparen. Nach der Befüllung steht an der Schnittstelle zum Durchfluß am Schaltventil ohne andere Zwischenvolumina, d.h. ohne sogenanntes Totvolumen, die Lösung der zuletzt befüllten Probenschleife an. Das bedeutet, daß die Meßkammer mit mehreren unterschiedlichen Lösungen aus den einzelnen Probenschleifen über die Wahlventile nacheinander, unabhängig voneinander und totvolumenfrei im Durchfluß beaufschlagbar ist, und ohne daß eine beliebige Probenschleife vollständig ausgespült bzw. der Fluß durch diese in eine andere Schleife geleitet werden muß. Damit ist eine variable, instantane, praktisch durchmischungsfreie und probensparende schrittweise Konzentrationsänderung gewährleistet. Zudem kann jeweils die Konzentration des Analyten in der Lösung in der Meßkammer in erster Näherung konstant gehalten werden, um eine Verarmung zu verhindern. Vorzugsweise ist in jeder Probenschleife eine Lösung unterschiedlicher Konzentration vorhanden, um Messungen mit unterschiedlichen Konzentrationen auch unmittelbar hintereinander ausführen zu können oder durch die gezielte Ansteuerung von Probenschleifen eine Zwischenkonzentration zwischen den Werten in den einzelnen Probenschleifen zu erhalten. Die Zahl der Probenschleifen ist praktisch zwar nicht begrenzt; zwecks höherer Flexibilität kann jedoch die o.g. gesamte Anordnung mitsamt des Schaltventils z.B. zwischen zwei im Durchfluß positionierten Zweifach-Wahlventilen ohne weiteres dupliziert werden. So kann der eine Probenschleifensatz abgearbeitet werden, während der andere Satz befüllt wird und praktisch totvolumenfrei in den Fluß geschaltet werden kann, noch bevor die letzte Schleife des ersten Satzes vollständig ausgespült worden ist.

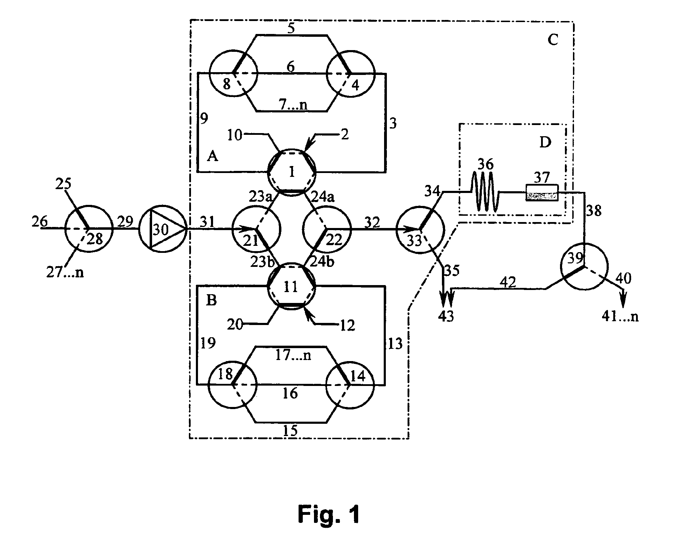

Eine Ausgestaltungsmöglichkeit der Probenaufgabe z.B. in einem Durchfluß-Meßsystem ist in Figur 1 wiedergegeben, die im folgenden beschrieben ist.

Mit Ausnahme der Pumpe 30, die z.B. eine Spritzenpumpe oder eine peristaltische Pumpe sein kann, symbolisieren alle übrigen Kreise Ventile, die wie hier beschrieben ausgelegt sein können. Die Ventile 1 und 11 sind Schaltventile, mit denen je nach Schaltung der beiden Schaltpositionen die entsprechenden Ein- bzw. Auslässe der Ventile verbunden werden; wobei in Figur 1 die aktuellen Verbindungen fett gezeichnet dargestellt sind und die alternativen Verbindungen gestrichelt. Die Ventile 4, 8, 14, 18, 28 und wenn gewünscht auch 21, 22, 33 und 39 sind Wahlventile, mit denen ein zentraler Anschluß, der mit normaler Linienbreite dargestellt ist, mit einem von beliebig vielen anderen Anschlüssen verbunden werden kann. Die Ventile 21, 22, 33 und 39 sind hier beispielhaft als Zweifach-Wahlventile gezeigt. Alle Ventilpositionen können manuell oder automatisch und auch vorprogrammiert mit z.B. elektrischem oder Druck-Antrieb gestellt werden. Alle Ventile sind vorzugsweise im Typ der in der Chromatographie verwendeten Drehventile ausgelegt, z.B. in volumenarmer Mikroausführung, können aber auch z.B. als Magnetventile ausgelegt werden. 36 ist eine beliebig dimensionierbare Temperierungs-Kapillare, 37 ist die Interaktions-Detektionseinheit, kurz Detektor, z.B. vom Typ eines Affinitäts-Biosensors. Alle mit normaler durchgezogener Linie angezeigten Verbindungen bestehen aus Kapillaren, gefertigt z.B. aus Tefzel, PE, PEEK oder Edelstahl, mit beliebigen Dimensionen bezüglich Innendurchmesser / Außendurchmesser / Länge, z.B. 1 mm $/^1/_{16}$ " / 0,5 m für die Verbindung 31. Eine beliebige Lösung, aus Vorräten 25 bis 27 oder folgenden n ausgewählt, wird mittels der Pumpe 30 über Ventil 33 entweder für Waschzwecke in den Abfall 43 oder durch den Detektor 37 gepumpt und über Ventil 39 in den Abfall 43 geleitet. Aus dem Detektor 37 ausgespülte Lösungen von weiterem Interesse können über das Ventil 39 im Sammler 41 gesammelt, in mehreren Gefäßen n fraktioniert aufgefangen oder mit anderen Techniken, z.B. Massenspektrometrie, direkt gekoppelt werden. Die in C enthaltenen Komponenten, mit Strich-Punkt umrahmt, können gemeinsam temperiert werden, z.B. mittels eines hier nicht gezeigten Peltier-Elementes.

Das erfindungsgemäß Besondere dieses Teils ist die Art der Probenaufgabe, die für eine repetitive Probenaufgabe und besonders für die Multischritt-Kinetik von Vorteil ist. Die Komponenten 1 bis 10 sind als Probenaufgabe A zusammengefaßt und über Ventil 1 entweder direkt in Verbindung 31 oder beliebig oft dupliziert über die Ventile 21 und 22 in die Verbindungen 31/32 einschaltbar; dargestellt ist hier eine Duplizierung von A, mit analoger Probenaufgabe B zusammengefaßt. In A befindet sich Ventil 1 in der Position Ladung für die Probenschleifen 5 bis 7, die über Probeneinlaß 2 entweder manuell oder mittels eines Probengebers automatisch mit beliebigen Lösungen, d.h. auch mit Analytlösungen jeweils fallender Konzentration, befüllt werden können, wobei die verdrängten Lösungen in Abfall 10 gespült werden. Minimal-Volumina, z.B. für Mikro- oder Test-Analysen, lassen sich realisieren, indem lediglich Verbindung 3 teilweise befüllt wird. Theoretisch unbegrenzte Maximal-Volumina einer Lösung, z.B. für sogenannte Präparationen mit besonders großen Sensoroberflächen, lassen sich realisieren, indem alle Probenschleifen mit derselben Lösung befüllt werden.

Nach jeweils synchronem Schalten der Ventile 4 und 8 können die Probenschleifen 5 bis 7 nacheinander und unabhängig voneinander sowie jeweils vollständig oder zwecks Proben-Ersparnis nur teilweise befüllt werden, und zwar in jedem Fall "rückwärts", d.h. von ihrer späteren Auslaßseite her. Damit ist gewährleistet, daß, nach dem Schalten von Ventil 1 in die Position Fluß, an die Verbindung 24a totvolumenfrei die Lösung der letzten Befüllung ansteht und sich daran nach zeitlich beliebigem, schrittweisen und jeweils synchronen Schalten der Ventile 4 und 8 übergangslos und unabhängig voneinander die Lösungen der anderen Probenschleifen auf den Detektor 37 beaufschlagen lassen. Diese Beaufschlagung ist in B gezeigt, wo sich die Komponenten 11 bis 20 spiegelbildlich zu A in Position Fluß befinden. Durch bevorzugt synchrones Schalten der Ventile 1, 11, 21 und 22 können A und B wechselseitig in Position Fluß bzw. Ladung umgeschaltet werden, so daß sich A im Fluß befindet, während B befüllt wird bzw. umgekehrt. Alle Verbindungskapillaren und Schleifen... 3, 9, 13, 19, 23a,b, 24a,b, 29, 31, 32, 34, 35, 38, 40, 42 und 5, 6, 7, 15, 16, 17 können je nach Anwendung in ihren Dimensionen besonders optimiert ausgelegt werden. In 24a und/oder 24b befindet sich normalerweise Spül- oder sogenannter Lauf-Puffer vom letzten Spül- oder Analysengang. Bei Bedarf können diese Verbindungskapillaren auch höhervolumiger ausgestaltet werden, um nach der Beaufschlagung der Lösungen aus B bzw. A und der gleichzeitigen Umschaltung der Lösungen aus A bzw. B in die Position Fluß zunächst eine Wasch- oder Dissoziationsphase in dem Detektor 37 durchzuführen, deren Dauer durch das Volumen von 24a bzw. 24b und der Flußrate bestimmt wird, was je nach Anwendung von Interesse sein kann. Die Anordnung ist darüber hinaus offen für die Positionierung weiterer Ventile und/oder Pumpen zwecks Waschung / Spülung beliebiger Abschnitte oder zwecks Einbringung weiterer Lösungen. So können z.B. B und/oder weitere Duplikate von B über Ventil 11 wahlweise z.B. in die Verbindungen 3, 5, 6, 7 oder 32 positioniert werden.

Desweiteren wird durch ein häufiges Schalten bestimmter, je nach Anwendung unterschiedlicher Ventile, eine Mischung von Lösungen ermöglicht, bei Bedarf auch durch Installation eines Flußteilers z.B. in Verbindung 31 anstelle von oder zusätzlich zu Ventil 21 und/oder einer sogenannten Mikro-Mischkammer z.B. in Verbindung 32 anstelle von oder zusätzlich zu Ventil 22. Diese Anordnung ergibt insgesamt eine größtmögliche Flexibilität der Lösungs-Beaufschlagung auf die Meßzelle. Ein Mischen der Vorräte 25 bis 27 oder mehr kann auch durch häufiges Schalten von Ventil 28 erreicht werden. Für Lösungen, die temperatursensitiv sind, können alle oder nur Teile der in C enthaltenen Komponenten, exklusive z.B. der in D enthaltenen mit Strich-Punkt-Punkt umrahmten Komponenten, gesondert temperiert werden, während die z.B. in D enthaltenen Komponenten ihrerseits gesondert auf eine Arbeitstemperatur temperiert werden.

**Index-Liste**

ass    Assoziation
diss    Dissoziation
i    i-ter Schritt
st    jeweiliger Startwert einer Assoziation oder Dissoziation

eq     jeweiliger Gleichgewichtswert einer Assoziation oder Dissoziation
net    Netto
$k_{on}$    Exponentialkoeffizient bei Assoziation
$k_{off}$   Exponentialkoeffizient bei Dissoziation

**Patentansprüche**

1. Analytisches Mess- und Auswerteverfahren für molekulare Interaktionen von chemischen Gleichgewichts-Bindungspaaren oder -Bindungssystemen, wobei ein erster Bindungspartner ("Ligand" L) mit einem in Lösung befindlichen zweiten Bindungspartner ("Analyt" A), der mit dem Liganden reversibel unter Komplexbildung reagiert, versetzt wird, Assoziations- und/oder Dissoziations-Kurven aufgenommen werden und die zeitliche Entwicklung der Komplexbildung $c_t$(LA) sowie deren Änderung $\dfrac{dc_t(LA)}{dt}$ erfasst werden,

**dadurch gekennzeichnet, dass**
zu bestimmten Zeitpunkten $t_i$ die Konzentration $c_{ti}$(A) einmal oder mehrmals schrittweise erhöht oder erniedrigt wird, wobei die Erhöhung bzw. Erniedrigung der Konzentration $c_{tj}$(A) jeweils zu einem beliebigen Zeitpunkt vor dem Erreichen der jeweiligen Gleichgewichtswerte der einzelnen Schritte durchgeführt wird, und der Reaktionsverlauf mittels eines mathematischen Annäherungsprogramms in 1. Näherung jeweils mit einer exponentiell verlaufenden Funktion R(t), die jeweils auf einen Gleichgewichtswert $R'_{eq,i}$ zuläuft, mit wenigstens einem Exponenten $k'_{on,i}$ oder

$k'_{off,i}$ beschrieben wird, wobei $\dfrac{dR(t)}{dt}$ die Assoziations- oder Dissoziationsrate des Analyten am Liganden angibt, um hieraus die Assoziations-Geschwindigkeitskonstante $k_{ass}$ und die Dissoziations-Geschwindigkeitskonstante $k_{diss}$ zu ermitteln.

2. Verfahren nach Anspruch 1,
**dadurch gekennzeichnet, dass**
aus den jeweiligen Differenzen zwischen den Assoziationsgleichgewichtswerten $R'_{eq,ass,i}$ und Assoziationsstartwerten $R'_{st,ass,i}$ oder den jeweiligen Differenzen zwischen den Dissoziationsgleichgewichtswerten $R'_{eq,diss,i}$ und Dissoziationsstartwerten $R'_{st,diss,i}$ und den jeweiligen Exponenten $k'_{on,i}$ oder $k'_{off,i}$ die jeweiligen initialen Assoziati-

ons- oder Dissoziationsraten $\left(\dfrac{dR_{ass,i}(t)}{dt}\right)_{t=0}$ bzw. $\left(\dfrac{dR_{diss,i}(t)}{dt}\right)_{t=0}$ und aus diesen Raten die Assoziations-

oder Dissoziationsgeschwindigkeitskonstante $k_{ass}$ bzw. $k_{diss}$ ermittelt werden.

3. Verfahren nach Anspruch 1,
**dadurch gekennzeichnet, dass**

die Dissoziationsgeschwindigkeitskonstante $k_{diss}$ aus den initialen Assoziationsraten $\left(\dfrac{dR_{ass,i}(t)}{dt}\right)_{t=0}$ und den jeweiligen Assoziationsgleichgewichtswerten $R'_{eq,ass,i}$ der Funktion R(t) und die Assoziationsgeschwindigkeitskonstante $k_{ass}$ aus den initialen Dissoziationsraten $\left(\dfrac{dR_{diss,i}(t)}{dt}\right)_{t=0}$ und den jeweiligen Dissoziationsgleichgewichtswerten $R'_{eq,diss,i}$ der Funktion R(t) ermittelt wird.

4. Verfahren nach Anspruch 1,
**dadurch gekennzeichnet, dass**
im Falle von jeweils konstanter Analytstartkonzentration $c_0$(A) bei Mehr-Schritt-Analysen die Werte für $k_{ass}$ und $k_{diss}$ aus der Beziehung zwischen $k'_{on}$ und der Analytstartkonzentration $c_0$(A) ermittelt werden.

5. Verfahren nach Anspruch 1,

**dadurch gekennzeichnet, dass**
die Assoziationsgeschwindigkeitskonstante $k_{ass}$ aus der Beziehung zwischen $k'_{on}$ und der Analytgleichgewichts-konzentration $c'_{eq,ass,i}(A)$ und die Dissoziationsgeschwindigkeitskonstante $k_{diss}$ aus der Beziehung zwischen $k'_{off}$ und dem Gleichgewichtswert $R'_{eq,diss,i}$ der Funktion $R(t)$ bei der Dissoziation ermittelt wird.

6. Verfahren nach einem der vorangehenden Ansprüche,
**dadurch gekennzeichnet, dass**
die Änderung der Analytkonzentration zur Halbwertszeit der Funktion $R_i(t)$ erfolgt.

7. Verfahren nach einem der vorangehenden Ansprüche,
**dadurch gekennzeichnet, dass**

die initiale Assoziations- oder Dissoziationsrate $\left(\dfrac{dR_{ass,i}(t)}{dt}\right)_{t=0}$ bzw. $\left(\dfrac{dR_{diss,i}(t)}{dt}\right)_{t=0}$ $(dR_{diss,i}(t)\ dt)_{t=0}$ einer linearen Beziehung zwischen $\dfrac{dR(t)}{dt}$ und $R(t)$ oder $(R(t) - R'_{st,diss,i})$ ermittelt wird.

8. Verfahren nach einem der vorangehenden Ansprüche,
**dadurch gekennzeichnet, dass**

die Ermittlung der initialen Raten $\left(\dfrac{dR_{ass,i}(t)}{dt}\right)_{t=0}$ bzw. $\left(\dfrac{dR_{diss,i}(t)}{dt}\right)_{t=0}$ unter der Annahme idealer Bedingungen erfolgt.

9. Verfahren nach Anspruch 8,
**dadurch gekennzeichnet, dass**

die Ermittlung der initialen Raten $\left(\dfrac{dR_{ass,i}(t)}{dt}\right)_{t=0}$ bzw. $\left(\dfrac{dR_{diss,i}(t)}{dt}\right)_{t=0}$ unter der Annahme erfolgt, dass bei der Assoziation keine Analytverarmung und bei der Dissoziation keine Analytanreicherung erfolgt.

10. Verfahren nach einem der vorangehenden Ansprüche,
**dadurch gekennzeichnet, dass**
die unbekannte Analytstartkonzentration $c_0(A)$ zu Beginn der Reaktion ermittelt wird.

11. Verfahren nach einem der vorangehenden Ansprüche,
**dadurch gekennzeichnet, dass**
das Verfahren mit bekannten Affinitätsbiosensoren und/oder Array-Systemen in Lösung und/oder zeitaufgelösten ELISA- oder RIA-Techniken oder anderen festphasengestützten Systemen durchgeführt wird.

12. Verfahren nach einem der vorangehenden Ansprüche,
**dadurch gekennzeichnet, dass**
in einem Küvettensystem die Analytkonzentration $c(A)$ der Lösung in der Messkammer durch kontinuierliche Entnahme eines Teils der Lösung und Zugabe einer Lösung mit einer entsprechenden Analytkonzentration $c(A)$ über eine oder mehrere Kanülen während des Assoziations- oder Dissoziationsvorgangs konstant gehalten wird.

13. Verfahren nach einem der vorangehenden Ansprüche,
**dadurch gekennzeichnet, dass**
bei konstanter Konzentration der Lösung in der Messkammer derjenige $c_0(A)$-Wert bestimmt wird, bei dem sich die Assoziationskurve oder ein Abschnitt davon mit der Dissoziationskurve oder einem Abschnitt davon in einem Punkt schneidet, dessen Wert 0,618 des gesamten oder des jeweiligen Teilwerts des Assoziations-Gleichgewichtswertes entspricht.

14. Verfahren nach Anspruch 13,
**dadurch gekennzeichnet, dass**

die Konzentration in der Messkammer mehrmals schrittweise geändert wird und die jeweiligen Startkonzentrationen des Analyten $c_{0,i}(A)$ ermittelt werden.

**15.** Verfahren nach einem der vorangehenden Ansprüche,
**dadurch gekennzeichnet, dass**
in einem idealen Durchflusssystem bei jeweils konstant gehaltener Analytstartkonzentration $c_{0,i}(A)$ während einer Assoziationsphase die Dissoziations-Gleichgewichtskonstante $K_D = k_{diss}/k_{ass}$ als diejenige Analytstartkonzentration $c_{0,i}(A)$ ermittelt wird, mit der der ermittelte Gleichgewichtswert gleich der Hälfte der maximal möglichen Reaktion $R_{max}$ auf die Zugabe des Analyten im Überschuss wird.

**16.** Vorrichtung zur Durchführung des Verfahrens nach einem der vorhergehenden Ansprüche, mit einer Sensoroberfläche zur Bindung des in Lösung befindlichen Analyten am Liganden, mit einer Meßkammer auf der Sensoroberfläche, die mit dem Analyt in Lösung befüllt ist, mit jeweils einer Kanüle zur Zugabe und/oder Herausnahme der Lösung und/oder einer Spülflüssigkeit, mit einer Vorrichtung zur Durchmischung der Lösung in der Meßkammer
**dadurch gekennzeichnet, dass**
die Lösungen in der Meßkammer zumindest teilweise in vorbestimmter Menge instantan mehrmals gegen Lösungen anderer Konzentration austauschbar sind, wobei das Volumen der Lösung konstant gehalten ist und wobei mehrere Probenschleifen vorhanden sind, deren Inhalt entgegen seiner Befüllrichtung und unabhängig voneinander in die Meßkammer einbringbar ist, so dass die Messkammer mit mehreren unterschiedlichen Lösungen aus den einzelnen Probenschleifen nacheinander, unabhängig voneinander und totvolumenfrei im Durchfluss beaufschlagbar ist.

**17.** Vorrichtung nach Anspruch 16,
**dadurch gekennzeichnet, dass**
der Austausch der Lösungen in der Meßkammer über eine oder mehrere Kanülen erfolgt.

**18.** Vorrichtung nach Anspruch 16 oder 17,
**dadurch gekennzeichnet, dass**
die Kanülen getrennt an der Meßkammer angeordnet sind oder die eine koaxial innerhalb der anderen.

**19.** Vorrichtung nach einem der Ansprüche 16 bis 18,
**dadurch gekennzeichnet, dass**
eine oder mehrere Pumpen zur Dosierung der Menge der auszutauschenden Lösungen an den Kanülen vorgesehen sind.

**20.** Vorrichtung nach einem der Ansprüche 16 bis 19,
**dadurch gekennzeichnet, dass**
über mehrere Kanülen die Lösung in der Meßkammer durch zumindest teilweise Absaugung und wieder Zugabe in die Meßkammer durchmischt wird.

## Claims

**1.** An analytical measuring and evaluation process for molecular interactions of chemical equilibrium binding couples and binding systems, wherein a first binding partner ("ligand" L) reversibly reacts with a second binding partner ("analyte" A) mixed in a solution, forming a complex compound, wherein the association and/or disassociation curves are being recorded and the temporal development of the complex forming $c_t(LA)$ and its derivative $\dfrac{dc_t(LA)}{dt}$ are recorded,
**characterized in that**
at certain points in time $t_i$ the concentration $c_{ti}(A)$ is increased or decreased in increments once or several times, wherein the increase or decrease of the concentration $c_{ti}(A)$ is performed at any time before reaching the respective equilibrium values of the particular steps and the reaction process is described with a mathematical approximation program, in a first approximation with an exponential function R(t), which approaches an equilibrium value $R'_{eq,i}$ with at least one exponent $k'_{on,i}$ or $k'_{off,i}$, wherein $\dfrac{dR(t)}{dt}$ provides the association or disassociation rate of the analyte at the ligand, in order to determine the association velocity constant $K_{ass}$ and the disassociation constant $k_{diss}$.

2. A process according to claim 1,
**characterized in that**
from the respective differentials between the association equilibrium values $R'_{eq,ass,i}$ and the association start values $R'_{st,ass,i}$ or the respective differentials between the disassociation equilibrium values $R'_{eq,diss,i}$ and the disassociation start values $R'_{st,diss,i}$ and the respective exponents $K'_{on,i}$ or $k_{off,i}$ the respective initial association or disassociation rates $\left(\dfrac{dR_{ass,i}(t)}{dt}\right)_{t=0}$ or $\left(\dfrac{dR_{diss,i}(t)}{dt}\right)_{t=0}$ and from these rates the association or disassociation velocity constant $k_{ass}$ or $k_{diss}$ are determined.

3. A process according to claim 1,
**characterized in that**
the disassociation velocity constant $k_{diss}$, is determined from the initial association rates $\left(\dfrac{dR_{ass,i}(t)}{dt}\right)_{t=0}$ and the respective association equilibrium values $R'_{eq,ass,i}$ of the function R(t) and the association velocity constant $k_{ass}$ from the initial disassociation rates $\left(\dfrac{dR_{diss,i}(t)}{dt}\right)_{t=0}$ and the respective disassociation equilibrium values $R'_{eq,diss,i}$ of the function R(t).

4. A process according to claim 1,
**characterized in that**
in case of a respectively constant initial analyte concentration $c_0(A)$ within multiple step analyses the values $k_{ass}$ and $k_{diss}$ are determined from the ratio of $k'_{on}$ and the analyte start concentration $c_0(A)$.

5. A process according to claim 1,
**characterized in that**
the association velocity constant $k_{ass}$ is determined from the ratio of $k'_{on}$ and the analyte equilibrium concentration $c_{eq,ass,i}(A)$ and the disassociation velocity constant $k_{diss}$ from the ratio between $k'_{off}$ and the equilibrium value $R'_{eq,diss,i}$ of the function R(t) during the disassociation.

6. A process according to one of the preceding claims,
**characterized in that**
the change of the analyte concentration happens at the half life of the function $R_i(t)$.

7. A process according to one of the preceding claims,
**characterized in that**
the initial association or disassociation rate $\left(\dfrac{dR_{ass,i}(t)}{dt}\right)_{t=0}$ or $\left(\dfrac{dR_{diss,i}(t)}{dt}\right)_{t=0}$ is determined from a linear relationship between $\dfrac{dR(t)}{dt}$ and R(t) or (R(t) - $R'_{st,diss,i}$).

8. A process according to one of the preceding claims,
**characterized in that**
the determination of the initial rates $\left(\dfrac{dR_{ass,i}(t)}{dt}\right)_{t=0}$ or $\left(\dfrac{dR_{diss,i}(t)}{dt}\right)_{t=0}$ is performed under the assumption of ideal conditions.

9. A process according to claim 8, **characterized in that**

the determination of the initial rates $\left(\dfrac{dR_{ass,i}(t)}{dt}\right)_{t=0}$ or $\left(\dfrac{dR_{diss,i}(t)}{dt}\right)_{t=0}$ is performed under the assumption, that during the association no analyte reduction and during the disassociation no analyte increase occurs.

10. A process according to one of the preceding claims,
**characterized in that**
the unknown analyte concentration $c_0(A)$ is determined at the beginning of the reaction.

11. A process according to one of the preceding claims,
**characterized in that**
the process is performed with known affinity biosensors and/or array systems in a solution and/or time based ELISA or RIA techniques or other solid phase based systems.

12. A process according to one of the preceding claims,
**characterized in that**
in a cuvette system the analyte concentration $c(A)$ of the solution in the measuring chamber is kept constant through continuous subtraction of a part of the solution and addition of a solution with the corresponding analyte concentration $c(A)$ through one or more canules during the association or disassociation process.

13. A process according to one of the preceding claims,
**characterized in that**
at a constant concentration of the solution in the measuring chamber the $c_0(A)$ value is determined, at which the association curve or a section thereof intersects with the disassociation curve or a section thereof in a point whose value 0.618 is equivalent to the whole or respective partial value of the association equilibrium value.

14. A process according to claim 13,
**characterized in that**
the concentration in the measuring chamber is changed incrementally several times and the respective start concentration of the analyte $c_{0,i}(A)$ is determined.

15. A process according to one of the preceding claims,
**characterized in that**
in an ideal flow through system with the analyte concentration $c_{0,i}(A)$ kept constant during an association phase, the disassociation equilibrium constant $K_D = k_{diss}/k_{ass}$ is determined as the analyte start concentration $c_{0,i}(A)$, through which the determined equilibrium value equals half of the maximum possible reaction $R_{max}$ with respect to the excessive addition of the analyte.

16. A device for performing the process according to one of the preceding claims with a sensor surface for binding the analyte in solution at the ligand, with a measuring chamber on the sensor surface which is filled with the analyte in solution, with a respective canule for adding and/or subtracting solution and/or a flushing liquid, with a device for mixing the solution in the measuring chamber,
**characterized in that**
the solutions in the measuring chamber are instantaneously exchangeable at least partially in a predetermined quantity, with solutions with other concentrations, wherein the volume of the solution is kept constant and wherein several sample loops are present, whose contents can be independently induced into the measuring chamber against their filling direction, so that the measuring chamber can be filled with several different solutions from the particular sample loops in sequence, independent from each other and without dead volume.

17. A device according to claim 16,
**characterized in that**
the replacement of the solutions in the measuring chamber is performed through several canules.

18. A device according to claim 16 or 17,
**characterized in that**
the canules are separately located at the measuring chamber or one is coaxial inside the other.

**19.** A device according to one of the claims 16-18
**characterized in that**
one or several pumps for dosing the quantity of solution to be replaced are provided at the canules.

**20.** A device according to one of the claims 16-19
**characterized in that**
the solution in the measuring chamber is mixed through several canules through at least partial subtraction and addition in the measuring chamber.

## Revendications

**1.** Procédé analytique de mesure et d'évaluation pour des interactions moléculaires de paires de liaison chimiques ou systèmes de liaison équilibrés, un premier partenaire de liaison ("ligand" L) mélangé à un second partenaire de liaison se trouvant dans une solution ("Analyte" A), qui réagit avec le ligand de manière réversible en formant un complexe, des courbes d'association et /ou de dissociation étant relevées et l'évolution temporelle de la formation du complexe $c_t$(LA) ainsi que sa modification $\dfrac{dc_t(LA)}{dt}$ étant enregistrées,

**caractérisé**
**en ce qu'** à des moments déterminés $t_i$, la concentration $c_{ti}$(A) est augmentée ou abaissée une fois ou plusieurs fois, l'augmentation respectivement l'abaissement de la concentration $c_{ti}$(A) étant effectués respectivement à n'importe quel moment avant d'atteindre les valeurs d'équilibre respectives de chacune des étapes et le déroulement de la réaction est décrit au moyen d'un programme mathématique d'approximation dans une première approximation avec une fonction R(t) évoluant exponentiellement, qui aboutit respectivement à une valeur d'équilibre R'$_{eq,i}$ avec au moins un exposant k'$_{on,i}$ ou k'$_{off,i}$, $\dfrac{dR(t)}{dt}$ indiquant les taux d'association et de dissociation de l'analyte sur le ligand, pour en déterminer les constantes d'équilibre d'association k$_{ass}$ et les constantes d'équilibre de dissociation k$_{diss}$.

**2.** Procédé selon la revendication 1,
**caractérisé**

**en ce que** les débits respectifs d'association ou de dissociation initiaux $\left(\dfrac{dR_{ass,i}(t)}{dt}\right)_{t=0}$ respectivement

$\left(\dfrac{dR_{diss,i}(t)}{dt}\right)_{t=0}$ sont déterminés à partir des différences respectives entre les valeurs d'équilibre d'association R'$_{eq,ass,i}$ et les valeurs initiales d'association R'$_{st,ass,i}$ ou à partir des différences entre les valeurs d'équilibre d'association R'$_{eq,aiss,i}$ et les valeurs initiales d'association R'$_{st,diss,i}$ et les exposants respectifs k'$_{on,i}$ ou k'$_{off,i}$, et les constantes d'équilibre d'association k$_{ass}$ respectivement de dissociation k$_{diss}$ sont déterminées à partir de ces taux.

**3.** Procédé selon la revendication 1,
**caractérisé**
**en ce que** la constante de vitesse de dissociation k$_{diss}$ est déterminée à partir des taux initiaux d' association $\left(\dfrac{dR_{ass,i}(t)}{dt}\right)_{t=0}$ et des valeurs respectives d'équilibre d'association R'$_{eq,ass,i}$ de la fonction R(t) et la constante de vitesse d'association k$_{ass}$ est déterminée á partir des taux initiaux d'association $\left(\dfrac{dR_{diss,i}(t)}{dt}\right)_{t=0}$ et des valeurs respectives d'équilibre de dissociation R'$_{eq,diss,i}$ de la fonction R(t).

**4.** Procédé selon la revendication 1,
**caractérisé en ce que**
dans le cas d'une concentration de départ d'analyte respectivement constante $c_0(A)$ pour des analyses en plusieurs étapes, les valeurs pour $k_{ass}$ et $k_{diss}$ sont déduites de la relation entre $k'_{on}$ et de la concentration de départ d'analyte $c_0(A)$.

**5.** Procédé selon la revendication 1,
**caractérisé en ce que**
la constante de vitesse d'association $k_{ass}$ résulte du rapport entre $k'_{on}$ et la concentration d'équilibre d'association $c'_{eq,ass,i}(A)$ et la constante de vitesse de dissociation $k_{diss}$ résulte du rapport entre $k'_{off}$ et la valeur d'équilibre $R'_{eq,diss,i}$ de la fonction $R(t)$ lors de la dissociation.

**6.** Procédé selon l'une des revendications précédentes,
**caractérisé en ce que**
la modification de la concentration d'analyte s'effectue à la période de demi-valeur de la fonction $R_i(t)$.

**7.** Procédé selon l'une des revendications précédentes,
**caractérisé en ce que**
les taux initiaux d'association et de dissociation $\left(\dfrac{dR_{ass,i}(t)}{dt}\right)_{t=0}$ respectivement $\left(\dfrac{dR_{diss,i}(t)}{dt}\right)_{t=0}$ sont déterminés à partir d'un rapport linéaire entre $\dfrac{dR(t)}{dt}$ et $R(t)$ ou $(R(t) - R'_{st,diss,i})$.

**8.** Procédé selon l'une des revendications précédentes,
**caractérisé en ce que**
la détermination des taux initiaux $\left(\dfrac{dR_{ass,i}(t)}{dt}\right)_{t=0}$ respectivement $\left(\dfrac{dR_{diss,i}(t)}{dt}\right)_{t=0}$ s'effectue dans l'hypothèse de conditions idéales.

**9.** Procédé selon la revendication 8,
**caractérisé en ce que**
la détermination des taux initiaux $\left(\dfrac{dR_{ass,i}(t)}{dt}\right)_{t=0}$ respectivement $\left(\dfrac{dR_{diss,i}(t)}{dt}\right)_{t=0}$ s'effectue dans l'hypothèse que lors de l'association, il ne se produit aucun appauvrissement et lors de la dissociation aucun enrichissement d'analyte

**10.** Procédé selon l'une des revendications précédentes,
**caractérisé en ce que**
la concentration d'analyte inconnue $c_0(A)$ est déterminée au début de la réaction.

**11.** Procédé selon l'une des revendications précédentes,
**caractérisé en ce que**
le procédé est effectué avec des biocapteurs connus d'affinité à résolution temporelle et/ou des systèmes de matrices (Array) en solution et/ou par des techniques d'immunodosage telles que les dosages radio-immunologiques (**RIA**) et immuno-enzymatiques (**ELISA**) ou autres systèmes en phase solide.

**12.** Procédé selon l'une des revendications précédentes,
**caractérisé en ce que**
dans un système de cuvette, la concentration d'analyte $c(A)$ de la solution dans la chambre de mesure est maintenue constante par prélèvement continu d'une partie de la solution et ajout d'une solution contenant une concentration correspondante d'analyte $c(A)$ par une ou plusieurs canules pendant l'opération d'association ou de dissociation.

**13.** Procédé selon l'une des revendications précédentes,
**caractérisé en ce**
**qu'**en présence d'une concentration constante de la solution dans la chambre de mesure, il est déterminé la valeur $c_0(A)$, à laquelle la courbe d'association ou une section se recoupe avec la courbe de dissociation ou une section en un point d'intersection, dont la valeur correspond à 0,618 de l'ensemble ou de la valeur partielle respective de la valeur d'équilibre.

**14.** Procédé selon la revendication 13,
**caractérisé en ce que**
la concentration dans la chambre de mesure est modifiée plusieurs fois progressivement et les concentration respective de départ de l'analyte $c_{0,i}(A)$ sont déterminées.

**15.** Procédé selon l'une des revendications précédentes, **caractérisé en ce que** dans un système idéal d'écoulement en présence d'une concentration de départ d'analyte maintenue constante $c_{0,i}(A)$ pendant une phase d'association, la constante d'équilibre de dissociation KD = $k_{diss}/k_{ass}$ est déterminée en tant que concentration de départ d'analyte $c_{0,i}(A)$ à laquelle la valeur déterminée d'équilibre égale à la moitié de la réaction maximale possible Rmax à l'ajout de l'analyte devient excédentaire.

**16.** Dispositif de mise en oeuvre du procédé selon l'une des revendications précédentes comprenant une surface de capteur pour la liaison de l'analyte, se trouvant en solution, au ligand, comprenant sur la surface de capteur une chambre de mesure qui est remplie de l'analyte en solution, comprenant respectivement une canule pour l'ajout ou le prélèvement de la solution et/ou d'un liquide de rinçage, comprenant un dispositif pour le mélange de la solution dans la chambre de mesure,
**caractérisé en ce**
les solutions sont échangeables dans au moins dans la chambre de mesure partiellement en quantité prédéterminée instantanément plusieurs fois contre des solutions d'une autre concentration, le volume de la solution étant maintenu constant et plusieurs boucles d'échantillon étant présentes, dont le contenu peut être introduit dans la chambre de mesure dans le sens contraire à la direction de remplissage et indépendamment les uns des autres de sorte que la chambre de mesure peut être alimentée de plusieurs solutions provenant des différentes boucles d'échantillon les unes après les autres indépendamment les uns des autres et sans volume mort dans le débit.

**17.** Dispositif selon la revendication 16, **caractérisé en ce que** l'échange des solutions dans la chambre de mesure s'effectue par une ou plusieurs canules.

**18.** Dispositif selon la revendication 16 ou 17, **caractérisé en ce que** les canules sont disposées séparément sur la chambre de mesure ou coaxialement l'une dans l'autre.

**19.** Dispositif selon l'une des revendications 16 à 18,
**caractérisé en ce qu'**
une ou plusieurs pompes sont prévues sur les canules pour le dosage de la quantité des solutions à échanger.

**20.** Dispositif selon l'une des revendications 16 à 19,
**caractérisé en ce que**
la solution est mélangée par plusieurs canules dans la chambre de mesure par au moins aspiration partielle et réintroduction dans la chambre de mesure.

**Fig. 1**